# EUROPEAN PATENT SPECIFICATION

(11) **EP 2 879 496 B1**
(45) Date of publication and mention of the grant of the patent: **22.08.2018**
(21) Application number: 13747589.3
(22) Date of filing: 30.07.2013
(51) Int. Cl.: A01N 43/40, A01N 57/20, A01N 43/16, A01N 37/44, A01P 1/00, A01P 3/00, A61K 8/49, C09D 5/14, A61Q 5/00, A61Q 5/02, A61Q 19/10

(54) **COMPOSITION AND METHOD FOR PREVENTING DISCOLORATION OF PYRITHIONE CONTAINING COMPOSITIONS**
ZUSAMMENSETZUNG UND VERFAHREN ZUR VERHINDERUNG EINER VERFÄRBUNG VON PYRITHIONEZUSAMMENSETZUNGEN
COMPOSITION ET PROCÉDÉ POUR PRÉVENIR LA DÉCOLORATION D'UNE COMPOSITION CONTENANT DE PYRITHIONE

(30) Priority: 31.07.2012 US 201261677704 P
(43) Date of publication of application: 10.06.2015
(73) Proprietor: Arch Chemicals, Inc., Atlanta, GA 30328 (US)
(72) Inventor: POLSON, George, Jasper, Georgia 30143 (US); JOURDEN, Jody, Atlanta, Georgia 30324 (US); TAYLOR, Denise B., Cumming, Georgia 30040 (US); JI, Yongcheng, Alpharetta, Georgia 30005 (US)
(74) Representative: Rummler, Felix
(86) International application number: PCT/US2013/052688
(87) International publication number: WO 2014/022369

(56) References cited:
- WO-A2-2011/056667
- GB-A- 2 109 551
- JP-A- 2002 020 207
- US-A- 4 301 162
- US-A- 4 818 436
- US-A- 4 935 061
- US-A1- 2012 015 986
- G. C.S. Manku: "T H E FORMATION CONSTANTS OF SOME METAL COMPLEXES WITH 3-ACETYL-4-HYDROXYCOUMARIN, DEHYDROACETIC ACID, AND THEIR OXIMES [Manuscript", Aust. J. Chem, vol. 24, 1 January 1971 (1971-01-01), pages 925-934, XP055443866,

## Description

### FIELD OF THE INVENTION

The present invention, relates to a composition containing a pyrithione compound and a pyranone compound and a method of reducing discoloration of compositions containing pyrithione compounds as defined in claims.

### BACKGROUND OF THE INVENTION

Metal salts of pyrithiones are known in the art to have biocidal activity and have been widely used as fungicides and bacteriocides. In particular, polyvalent metal salts of pyrithione (also known as 1-hydroxy-2-pyridinethione; 2-pyridinethiol-1-oxide; 2-pyridinethione; 2-mercaptopyridine-N-oxide; pyridinethione; and pyridinethione-N-oxide) are known to be effective biocidal agents. As such, polyvalent metal salts of pyrithione compounds have been used in a wide variety of compositions including personal care compositions, such as cosmetics, and antidandruff shampoos; material protection products, such as paints, adhesives, caulks and sealants; plastics and metal working fluids. In addition, the use of polyvalent metal salts of pyrithione is generally growing.

One significant problem encountered in the manufacture of finished formulations (compositions) containing pyrithione compounds is the interaction of extraneous iron salts that may be introduced to the formulations from raw materials or processing equipment. In the presence of ferric ion, sodium pyrithione or zinc pyrithione-containing compositions tend to turn blue even though the ferric ion is present in mere trace amounts. This blue discoloration is undesirable for aesthetic reasons, as well as for functional reasons relating to unwanted color in the resulting composition. Since the aesthetics of shampoos, paints, adhesives, caulks and sealants normally require certain desirable colors, and since the formulators of such products go to great lengths to achieve specific color effects, any ingredient which causes the formulation to vary much from a desired white or colorless hue may make the colorant formulators' task very difficult. More specifically, when attempting to utilize pyrithione as an antimicrobial agent in fully-formulated shampoos, water-based paints, paint bases (i.e., the partially formulated paint before pigment addition), adhesives, caulks and sealants, an unwanted color from the additive can adversely affect the color of the formulated product or change the intended color of the finished product. As a result, this undesired color might make the formulations undesirable for their intended use.

In the past, various solutions to the blue discoloration problem have been proposed. By way of illustration, U.S. Pat. Nos. 6,096,122 and 5,939,203 and 5,883,154 and 5,562,995 all disclose solutions to the above problem by addition of inorganic compounds of zinc. U.S. Patents 4,957,658 and 4,818,436 disclose solutions to the above-discussed discoloration problem by adding to the paint or functional fluid an alkali metal or alkaline earth metal salt of 1-hydroxyethane-1,1-diphosphonic acid. Although these patents propose solutions to the discoloration problem, these solutions are not always as cost effective or do not work at a broad pH range (4-11). In some situations, these solutions may not be as permanent as might be desired.

US 2012/015986 A1 describes a method of inhibiting discoloration of products containing pyrithione or a salt thereof with a discoloration inhibitor containing dehydroacetic acid in free or salt form; US 4,301,162 discloses an antibacterial and antifungal composition comprising a mixture of (a) dehydroacetic acid or its alkali metal salts and (b) 2-pyridinethiol 1-oxide and its salts; JP 2002-020207 mentions an antimicrobial composition containing sodium dehydroacetate, sodium pyrithione and thiabendazole; WO 2011/056667 A2 discloses a method for topical treatment of rosacea comprising application of one or more iron chelators, e.g. a bispyrithione salt and kojic acid; and US 4,935,061 describes a process and composition for providing reduced discoloration of paints and paint bases containing a pyrithione using 1-hydroxyethane-1,1-diphosphoric acid.

There is a need in the art for an economical and environmentally friendly solution to the discoloration problem that will allow pyrithione compounds to be used in the presence of iron ions which will work in a broad pH range and uses relatively low levels additives which are generally considered to be safe.

### SUMMARY OF THE INVENTION

Generally stated, the present invention, as defined in the claims, provides a cost effective composition containing a pyrithione compound which has a reduced discoloration from the presence of iron ions in the composition.

In one embodiment of the present invention, provided is a biocidal composition containing a zinc pyrithione compound in an amount effective to provide biocidal properties to the composition and a pyranone compound and an iron co-chelator comprising a compound selected from the group consisting of hydroxyethylidene diphosphonic acid, ethylene diamine tetraacetic acid, salts of each of the proceeding compounds and mixtures thereof, wherein each of the pyranone compound and the iron co-chelator are present in an amount effective to reduce or eliminate discoloration of the biocidal composition due to the presence of an iron ion, and wherein the weight ratio of the pyranone compound to the iron co-chelator is from 0.01:10 to 10:0.01.

.

In another embodiment of the present invention, provided is the use for reducing discoloration of a zinc pyrithione-compound containing composition comprising adding a pyranone compound and an iron co-chelator to the zinc pyrithione-compound containing composition for complexing iron ions introduced to the composition from impurities present in raw materials used to make the composition or from the processing equipment, wherein the weight ratio of the pyranone compound to the iron co-chelator is from 0.01:10 to 10:0.01, wherein the iron co-chelator comprises a compound selected from the group consisting of hydroxyethylidene diphosphonic acid, ethylene diamine tetraacetic acid, salts of each of the proceeding compounds and mixtures thereof.

In a particular embodiment of the present invention, the present invention is directed to a soap composition which contains the biocidal composition as defined in the claims.

By providing the pyrithione containing composition with a pyranone and the iron co-chelator as defined in claims, the effects of the presence of iron ions in the composition with respect to discoloration are reduced or even eliminated.

### DEFINITIONS

It should be noted that, when employed in the present disclosure, the terms "comprises", "comprising" and other derivatives from the root term "comprise" are intended to be open-ended terms that specify the presence of any stated features, elements, integers, steps, or components, and are not intended to preclude the presence or addition of one or more other features, elements, integers, steps, components, or groups thereof.

As used herein, the term "zinc-pyrithione containing composition" is intended to mean a composition containing a pyrithione zinc salt.

### DETAILED DESCRIPTION OF THE INVENTION

The present invention relates to the use comprising adding a pyranone compound and an iron co-chelator as defined in claims to prevent chelation of the zinc pyrithione-compound with extraneous sources of iron from raw materials or processing equipment, thereby preventing aesthetically undesirable colors in finished products, such as a blue-black colors in finished products. The finished products are prepared from a biocidal composition containing the zinc-pyrithione salt. Since zinc-pyrithione itself is a very good chelator of free iron species, there needs to be another chelator that can competitively chelate with free iron as well as minimize the ionization of pyrithione complexes. The present invention as defined in the claims relates to both the composition containing the zinc-pyrithione compounds as well as the use for reducing discoloration of a composition of containing a pyrithione compound.

Pyrithione compounds (also known as 1-hydroxy-2-pyridinethione; 2-pyridinethiol-1-oxide; 2-pyridinethione; 2-mercaptopyridine-N-oxide; pyridinethione; and pyridinethione-N-oxide) are known in the art and are known to be effective biocidal agents. In particular, polyvalent metal salts of pyrithione compounds are generally used as biocidal agents. Synthesis of polyvalent pyrithione salts are described in U.S. Pat. No. 2,809,971 to Berstein et al., each hereby incorporated by reference. Other patents disclosing similar compounds and processes for making them include, for example, U.S. Patent 2,786,847; U.S. Patent 3,589,999; U.S. Patent 3,590,035; U.S. Patent 3,773,770. Complexes of 1-hydroxy-2-pyridinethione can be found in the publication by Robinson, M. A. Journal of Inorganic and Nuclear Chemistry (1964), 26(7), 1277-81. Suitable metal salts or complexes of pyrithiones, such as zinc (in the invention embodiments), copper, bismuth, tin, cadmium, magnesium, aluminum, and zirconium may be used in the composition, except for zinc pyrithione which is part of the invention for reference purposes. Generally, zinc salt of 1-hydroxy-2-pyridinethione, known in the art as zinc pyrithione, is the most widely used. Commercially available pyrithione salts suitable herein include zinc pyrithione available from Arch Chemicals, Inc.

It has been discovered that a class of pyranone compounds are very useful iron chelators and that pyranone compounds effectively competitively chelate with free iron as well as minimize the ionization of pyrithione complexes in biocidal compositions. By chelating the iron with the pyranone compound and the iron co-chelator as defined in claims the zinc pyrithione compound containing composition will tend not to be discolored by the formation of pyrithione complexes with the iron. Various examples of pyranone which are useful to prevent or reduce discoloration include, but are not limited to, pyranones of structure (I): 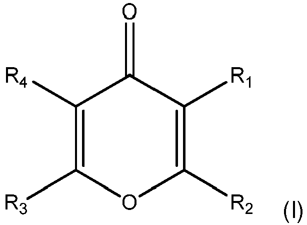 where R₁ R₂, R₃ and R₄ are each independently a H, OH, (C=O) alkyl group or alkyl group which may be substituted with an halogen, a hydroxyl group and the like.

Specific examples of pyranones which may be used in the present invention include 3-acetyl-6-methyl-2H-pyranone (dehydroacetic acid or DHA) having the structure (II): 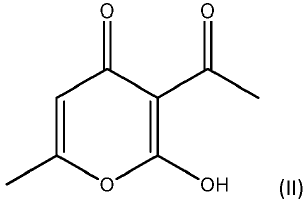 Kojic acid having the structure (III): 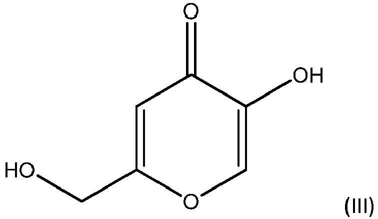 or Maltol having the structure (IV):

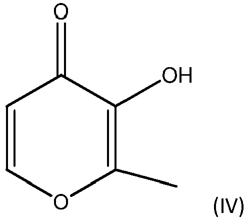

In addition, the pyranone compounds may be a salt of the pyranone. Suitable salts include metal salts, such as zinc salts, calcium salts, magnesium salts and sodium salts. Generally, zinc salts and sodium salts are used.

By adding the pyranone compound, or salt thereof and the iron co-chelator, or salt thereof to a composition containing a zinc pyrithione, it is believed that any iron ions present in the composition will chelate with the pyranone compound and the iron co-chelator will avoid the chelation of the iron ions with the zinc pyrithione, thereby reducing or avoiding the discoloration of the resulting composition containing the zinc pyrithione compound.

The iron ion co-chelators include hyroxyethylidene diphosphonic acid, ethylene diamine tetraacetic acid salts of each of the proceeding compounds or a mixture thereof. Suitable salts include, for example, zinc salts, calcium salts, magnesium salts or sodium salts of these co-chelators. It has been discovered that the addition of the co-chelator further helps reducing the discoloration of the resulting composition.

Generally, in a composition containing a zinc pyrithione compound, the composition may contain up to 10% by weight, based on the total weight of the composition of the zinc pyrithione compound as the effective amount. Higher weight percentages may be used, if necessary. Typically, the composition will contain from 0.001 % to 5.0 % by weight, based on the total weight of the composition, of the zinc pyrithione. In most applications the zinc pyrithione is present in an amount between 0.01% to 3% by weight, based on the total weight of the composition.

In a composition containing a pyrithione or a pyrithione complex, the composition may contain up to 10% by weight, based on the total composition, of the pyranone compound or pyranone salt as the effective amount. Higher weight percentages may be used, if necessary. Typically, the composition will contain from 0.001 % to 5.0 % by weight, based on the total weight of the composition, of the pyranone or pyranone salt. In most applications, the pyranone or pyranone salt is present in an amount between 0.01% to 3% by weight, based on the total weight of the composition.

The iron co-chelator may be present in an amount up to 10% by weight, based on the total composition, as the effective amount. Higher weight percentages may be used if necessary. Typically, the composition will contain from 0.001 % to 5.0 % by weight, based on the total weight of the composition, of the iron co-chelator. In most applications, the iron co-chelator is present in an amount between 0.01% to 3% by weight, based on the total weight of the composition. The weight ratio of the pyranone compound or salt thereof to the iron co-chelator is in the range of 0.01 : 10 to 10: 0.01, more typically in the range of 0.01 : 1 to 1: 0.01.

The pyrithione-containing compositions of the present invention can be used in a wide variety of uses including, for example, a soap, a shampoo, a hand sanitizer, a deodorant, a metal working fluid, a wood preservative, a paint, a coating, or a plastic precursor. The balance of the composition, apart from the zinc pyrithione compound thereof, the pyranone compound or salt thereof, and/or the iron co-chelator are components which make the composition useful for its intended purpose. For example, a soap composition having biocidal properties will contain ingredients typically found in soap. These types of compositions are well known in the art.

An exemplary soap composition will generally contain a soap component, a pH adjusting agent, and other additives. Suitable soap components include, for example alkali metal or alkanol ammonium salts of alkane- or alkene monocarboxylic acids. Alkali metals suitable include, sodium, magnesium, potassium, calcium, and alkanol ammonium salts include mono-, di- and tri-ethanol ammonium cations. Combinations thereof are also suitable. Generally, soaps having the fatty acid distribution of coconut oil may provide the lower end of the broad molecular weight range. Those soaps having the fatty acid distribution of peanut or rapeseed oil, or their hydrogenated derivatives, may provide the upper end of the broad molecular weight range.

Generally, soaps having the fatty acid distribution of tallow and vegetable oil are used. More particularly, the vegetable oil is selected from the group consisting of palm oil, coconut oil, palm kernel oil, palm oil stearine, and hydrogenated rice bran oil, or mixtures thereof, since these are among the more readily available fats. Especially preferred are palm oil stearine, palm kernel oil, and/or coconut oil. The proportion of fatty acids having at least 12 carbon atoms in coconut oil soap is about 85%. This proportion will be greater when mixtures of coconut oil and fats such as tallow, palm oil, or non-tropical nut oils or fats are used, wherein the principal chain lengths are C₁₆ and higher.

Soaps may be made by the classic kettle boiling process or modern continuous soap manufacturing processes wherein natural fats and oils such as tallow or coconut oil or their equivalents are saponified with an alkali metal hydroxide using procedures well known to those skilled in the art. Alternatively, the soaps may be made by neutralizing fatty acids, such as lauric (C₁₂), myristic (C₁₄), palmitic (C₁₆), or stearic (C₁₈) acids with an alkali metal hydroxide or carbonate.

The pH of the present bar soap is greater than or equal to 10. Soap comprises a pH adjusting agent in a sufficient amount to attain the above mentioned pH. The pH adjusting agents useful for the present composition includes alkalizing agents. Suitable alkalizing agents include, for example, ammonia solution, triethanolamine, diethanolamine, monoethanolamine, potassium hydroxide, sodium hydroxide, sodium phosphate dibasic, soluble carbonate salts, ammonia solution, triethanolamine, diethanolamine, monoethanolamine, potassium hydroxide, sodium hydroxide, sodium phosphate dibasic, soluble carbonate salts and combinations thereof.

Other ingredients may be present in the soap, including surfactants, structurants, colorants, moisturizers, skin conditioners, brighteners, opacifiers, fragrances, perfumes, and other such additives conventionally added to soaps.

To add the pyranone compound or salt thereof to the zinc pyrithione containing composition, the pyranone compound or salt thereof is mixed with the components of the composition, including the zinc pyrithione compound. The pyranone compound or salt may be added as a solution, aqueous slurry, or as a solid.

### EXAMPLES

### Example 1

Solutions were prepared according to TABLE 1 in water with a final concentration of 0.5% zinc pyrithione (ZPT), 0.25% chelator or its Zn salt, and 125 ppm Fe(III) at a pH of 10-10.3. ZPT was weighed into 100 mL sample containers. To this was added 20 g of H₂O and the chelator or chelators as shown in TABLE 1. In TABLE 1, DHA is acetyl-6-methyl-2H-pyranone (dehydroacetic acid) (Geogard 111A), DHA-Zn is a zinc salt of acetyl-6-methyl-2H-pyranone, Wayhib-Zn is a commercial hyroxyethylidene diphosphonic acid (HEDP) zinc salt. Each of DHA, and DHA-Zn were added as solids. HEDP-Zn, which is a zinc salt of hyroxyethylidene diphosphonic acid (sold under the trade name Wayhib-Zn), were added as solutions in which the Zn-salt was pre-formed in an aqueous solution then added to the sample. The formulations were then sonicated for two minutes and then adjusted to a pH of approximately 10.0 using 1.0 M NaOH. The remaining amount of water was then added to each sample and the pH was adjusted again if needed. The samples were placed on a shaker for 30 minutes to ensure thorough mixing. Following the 30 minutes, 0.5 g of a FeCl₃ stock was added to give a final concentration of 125 ppm Fe(III) in each solution.

**TABLE 1 Samples 1, 2 and 3 are reference examples**

| | SAMPLE | **1** | **2** | **3** | **4** |
|---|---|---|---|---|---|
| wt% | ZPT | 0.50 | 0.50 | 0.50 | 0.50 |
| | DHA | | 0.25 | | |
| | DHA-Zn | | | 0.25 | 0.125 |
| | Wayhib-Zn | | | | 0.125 |
| | | | | | |
| g | ZPT(48%) | 0.42 | 0.42 | 0.42 | 0.42 |
| | DHA | | 0.10 | | |
| | DHA-Zn | | | 0.10 | 0.05 |
| | Wayhib-Zn (3.3%) | | | | 1.52 |
| | Fe (10000ppm stock) | 0.50 | 0.50 | 0.50 | 0.50 |
| | H2O | 39.08 | 38.98 | 38.98 | 37.52 |
| batch total (g) | | 40.00 | 40.00 | 40.00 | 40.00 |
| Final pH | | 10.30 | 10.06 | 10.08 | 10.15 |
| Color | | Green/Brown | Light Beige | Light Beige | Pale Yellow |

### Example 2

A melt and pour soap base containing the following ingredients coconut oil, palm oil, safflower oil, glycerin (kosher, of vegetable origin), purified water, sodium hydroxide (saponifying agent), sorbitol (moisturizer), sorbitan oleate (emulsifier), soy bean protein (conditioner), titanium dioxide (mineral whitener used in opaque soaps) was heated and melted. Combinations of ZPT with iron chelators were formulated into a melt and pour soap base and the resulting color of the compositions were compared to the melt and pour soap base as shown in Table 2. Samples A and B containing the formulation shown in the Table 2 show similar coloring as that of the control melt and pour soap base.

**TABLE 2**

| | | **Sample A** | **Sample B** |
|---|---|---|---|
| wt% | ZPT | 0.500 | 0.500 |
| | DHA-Zn | 0.125 | 1.000 |
| | HEDP-Zn | 0.125 | 0.500 |
| | | | |
| g | ZPT(48%) | 0.417 | 0.417 |
| | DHA-Zn | 0.050 | 0.400 |
| | HEDP-Zn (3.3%) | 1.563 | 6.250 |
| | Soap Base | 37.971 | 32.933 |

| | Fe(III) | 10 ppm | 125 ppm |
|---|---|---|---|
| batch total (g) | | 40.00 | 40.00 |
| Color | | White | White |

The soap composition is essentially the same color as the base without the ZPT and the chelator composition.

### Example 3

Samples of ZPT with iron chelators formulated into a melt and pour soap base used in Example 2. After melting of the soap base in a beaker, Fe(III) was added as a solution of FeCl₃ in water producing a light yellow color. This was followed by the addition of iron chelators and then finally ZPT in the amounts shown in Table 3. Samples were poured into vials after color changes had stopped. The composition of each sample is shown in TABLE 3, as is the resulting color of the soap when iron is present. Color was observed with the naked eye of the soap in the vials.

**TABLE 3 "Sample 1" is a reference example**

| | Sample 1 | Sample 2 | Sample 3 | Sample 4 |
|---|---|---|---|---|
| Amounts of components in Soap | 0.5% ZPT | 0.5% ZPT | 0.5% ZPT | 0.5% ZPT |
| | | 0.05% HEDP-Zn | 0.125% HEDP-Zn | 0.20% HEDP-Zn |
| | 125 ppm | 0.20% DHA-Zn | 0.125% DHA-Zn | 0.05% DHA-Zn |
| | Fe(III) | 125 ppm Fe(III) | 125 ppm Fe(III) | 125 ppm Fe(III) |
| Color of resulting soap | Grey with black spots | Light Grey | White | White with light orange specks |

As shown in TABLE 3, a series of formulations using DHA-Zn and HEDP-Zn combinations were formed. When using DHA-Zn as the chelator, increasing HEDP-Zn to DHA-Zn lessens the grey coloring. The combination of chelators improves the overall color of the soap composition, lessening the effect of iron ions on the pyrithione compound. However, as can be seen, the addition of the DHA and HEDP combination helps reduce the color form an unacceptable grey with black spots to a light grey, white with some specks of other colors. These colors are considered acceptable, versus the soap without the DHA-Zn and HEDP-Zn additives.

## Claims

1. The use for reducing discoloration of a zinc pyrithione-compound containing composition comprising adding a pyranone compound and an iron co-chelator to the zinc pyrithione-compound containing composition for complexing iron ions introduced to the composition from impurities present in raw materials used to make the composition or from the processing equipment, wherein the weight ratio of the pyranone compound to the iron co-chelator is from 0.01:10 to 10:0.01,
wherein the iron co-chelator comprises a compound selected from the group consisting of hydroxyethylidene diphosphonic acid, ethylene diamine tetraacetic acid, salts of each of the proceeding compounds and mixtures thereof.

2. The use according to claim 1, wherein the pyranone compound comprises 3-acetyl-6-methyl-2H-pyranone or a salt thereof.

3. The use according to claim 2, wherein the salt of 3-acetyl-6-methyl-2H-pyranone comprises a sodium salt or a zinc salt.

4. The use according to claim 2, wherein the 3-acetyl-6-methyl-2H-pyranone is added to the zinc pyrithione-containing composition as a solid or as an aqueous slurry.

5. The use according to claim 1, wherein the pyranone compound is added in a range of 0.001% to 5% by weight, based on the total weight of the composition.

6. The use according to claim 1, wherein the weight ratio of the pyranone compound to the iron co-chelator is in the range of 0.01: 1 to 1: 0.01.

7. The use according to claim 1, said zinc pyrithione compound containing composition comprising a zinc pyrithione in an amount between 0.001% to 5.0 % by weight of the composition.

8. The use according to any one of claims 1 to 7, wherein the zinc pyrithione compound containing composition is a soap, a shampoo, a hand sanitizer, a deodorant, a metal working fluid, a wood preservative, a paint, a coating, or a plastic precursor.

9. The use according to any one of claims 1 to 7 wherein the zinc pyrithione compound containing composition is used as biocidal composition.

10. The use according to claim 9 wherein the biocidal composition is a soap.

11. A biocidal composition comprising:
a zinc pyrithione;
a pyranone compound; and
an iron co-chelator comprising a compound selected from the group consisting of hydroxyethylidene diphosphonic acid, ethylene diamine tetraacetic acid, salts of each of the proceeding compounds and mixtures thereof,
wherein each of the pyranone compound and the iron co-chelator are present in an amount effective to reduce or eliminate discoloration of the biocidal composition due to the presence of an iron ion, and
wherein the weight ratio of the pyranone compound to the iron co-chelator is from 0.01:10 to 10:0.01.

12. The composition according to claim 11, wherein the weight ratio of the pyranone compound to the iron co-chelator is in the range of from 0.01:1 to 1:0.01 and wherein based on the weight of the composition, the zinc pyrithione comprises between 0.001% to 5.0% by weight, said pyranone compound comprises between 0.001 % to 5.0% by weight and said iron co-chelator comprises 0.001 % to 5.0 % by weight.

## Patentansprüche

1. Verwendung zum Reduzieren der Verfärbung einer Zinkpyrithionverbindung enthaltenden Zusammensetzung, umfassend das Zusetzen einer Pyranonverbindung und eines Eisen-co-chelators zu der Zinkpyrithionverbindung enthaltenden Zusammensetzung zum Komplexieren von Eisenionen, die in die Zusammensetzung aus Verunreinigungen, die in Rohmaterialien vorliegen, die zum Herstellen der Zusammensetzung verwendet werden, oder aus der Verarbeitungsvorrichtung eingeführt worden sind, wobei das Gewichtsverhältnis der Pyranonverbindung zu dem Eisen-co-chelator 0,01:10 bis 10:0,01 beträgt,
wobei der Eisen-co-chelator eine Verbindung umfasst ausgewählt aus der Gruppe bestehend aus Hydroxyethylidendiphosphonsäure, Ethylendiamintetraessigsäure, Salzen von jedem der vorstehenden Verbindungen und Mischungen davon.

2. Verwendung nach Anspruch 1, wobei die Pyranonverbindung 3-Acetyl-6-methyl-2H-pyranon oder ein Salz davon umfasst.

3. Verwendung nach Anspruch 2, wobei das Salz von 3-Acetyl-6-methyl-2H-pyranon ein Natriumsalz oder ein Zinksalz umfasst.

4. Verwendung nach Anspruch 2, wobei das 3-Acetyl-6-methyl-2H-pyranon der Zinkpyrithion enthaltenden Zusammensetzung als Feststoff oder als wässrige Aufschlämmung zugesetzt wird.

5. Verwendung nach Anspruch 1, wobei die Pyranonverbindung in einem Bereich von 0,001 bis 5 Gew.-%, auf das Gesamtgewicht der Zusammensetzung bezogen, zugesetzt wird.

6. Verwendung nach Anspruch 1, wobei das Gewichtsverhältnis der Pyranonverbindung zu dem Eisen-co-chelator im Bereich von 0,01:1 bis 1:0,01 liegt.

7. Verwendung nach Anspruch 1, wobei die Zinkpyrithionverbindung enthaltenden Zusammensetzung ein Zinkpyrithion in einer Menge von 0,001 bis 5,0 Gew.-%, auf die Zusammensetzung bezogen, umfasst.

8. Verwendung nach irgendeinem der Ansprüche 1 bis 7, wobei die Zinkpyrithionverbindung enthaltende Zusammensetzung eine Seife, ein Shampoo, ein Handreiniger, ein Deodorant, ein Metallbearbeitungsfluid, ein Holzkonservierungsmittel, ein Anstrichmittel, eine Beschichtung oder ein Kunststoffvorläufer ist.

9. Verwendung nach irgendeinem der Ansprüche 1 bis 7, wobei die Zinkpyrithionverbindung enthaltende Zusammensetzung als biozide Zusammensetzung verwendet wird.

10. Verwendung nach Anspruch 9, wobei die biozide Zusammensetzung eine Seife ist.

11. Biozide Zusammensetzung umfassend:
ein Zinkpyrithion;
eine Pyranonverbindung; und
einen Eisen-co-chelator, der eine Verbindung umfasst ausgewählt aus der Gruppe bestehend aus Hydroxyethylidendiphosphonsäure, Ethylendiamintetraessigsäure, Salzen von jedem der vorstehenden Verbindungen und Mischungen davon,
wobei jedes von der Pyranonverbindung und dem Eisen-co-chelator in einer Menge vorliegt, die zum Reduzieren oder Eliminieren von Verfärbung der bioziden Zusammensetzung aufgrund des Vorliegens eines Eisenions wirksam ist, und
wobei das Gewichtsverhältnis der Pyranonverbindung zu dem Eisen-co-chelator 0,01:10 bis 10:0,01 beträgt.

12. Zusammensetzung nach Anspruch 11, wobei das Gewichtsverhältnis der Pyranonverbindung zu dem Eisen-co-chelator im Bereich von 0,01:1 bis 1:0,01 liegt und wobei, auf das Gewicht der Zusammensetzung bezogen, das Zinkpyrithion 0,001 bis 5,0 Gew.-% umfasst, wobei die Pyranonverbindung 0,001 bis 5,0 Gew.-% umfasst und der Eisen-co-chelator 0,001 bis 5,0 Gew.-% umfasst.

## Revendications

1. Utilisation permettant de réduire la décoloration d'une composition contenant un composé de type pyrithione de zinc consistant à ajouter un composé de type pyranone et un fer co-chélateur à la composition comprenant un composé de type pyrithione de zinc permettant de complexer des ions fer introduits dans la composition à partir d'un pureté présente dans des matières premières utilisées pour préparer la composition ou à partir de l'équipement de traitement, dans laquelle le rapport massique du composé de type pyranone au fer co-chélateur est compris entre 0,01:10 et 10:0,0,1 dans laquelle le fer co-chélateur comprend un composé choisi parmi le groupe constitué de l'acide hydroxyéthylidène diphosphonique, de l'acide éthylène diamine tétraacétique, de sels de chacun des composés précédents et des mélanges de ceux-ci.

2. Utilisation selon la revendication 1, dans laquelle le composé de type pyranone comprend la 3-acétyl-6-méthyl-2H-pyranone ou un sel de celle-ci.

3. Utilisation selon la revendication 2, dans laquelle le sel de la 3-acétyl-6-méthyl-2H-pyranone comprend un sel de sodium ou un sel de zinc.

4. Utilisation selon la revendication 2, dans laquelle la 3-acétyl-6-méthyl-2H-pyranone est ajoutée à la composition comprenant de la pyrithione de zinc en tant qu'un solide ou en tant qu'une boue aqueuse.

5. Utilisation selon la revendication 1, dans laquelle le composé de type pyranone est ajouté dans une gamme comprise entre 0,001 % et 5,0 % en poids, sur la base du poids total de la composition.

6. Utilisation selon la revendication 1, dans laquelle le rapport massique du composé de type pyranone au fer co-chélateur se trouve dans la gamme comprise entre 0,01:1 et 1:0,01.

7. Utilisation selon la revendication 1, ladite composition contenant un composé de type pyrithione de zinc comprend une pyrithione de zinc dans une quantité comprise entre 0,001 % et 5,0 % en poids de la composition.

8. Utilisation selon l'une quelconque des revendications 1 à 7, dans laquelle la composition contenant un composé de type pyrithione de zinc est un savon, un shampooing, un désinfectant pour les mains, un déodorant, un fluide pour le travail des métaux, un agent de préservation du bois, une peinture, un revêtement, ou un précurseur plastique.

9. Utilisation selon l'une quelconque des revendications 1 à 7, dans laquelle la composition contenant un composé de type pyrithione de zinc est utilisée en tant que composition biocide.

10. Utilisation selon la revendication 9, dans laquelle la composition biocide est un savon.

11. Composition biocide comprenant :
une pyrithione de zinc ;
un fer co-chélateur comprenant un composé choisi parmi le groupe constitué de l'acide hydroxyéthylidène diphosphonique, de l'acide éthylène diamine tétraacétique, de sels de chacun des composés précédents et des mélanges de ceux-ci,
dans laquelle chacun du composé de type pyranone et du fer co-chélateur sont présents dans une quantité efficace pour réduire ou éliminer la décoloration de la composition biocide du fait de la présence d'un ion fer, et
dans laquelle le rapport massique du composé de type pyranone au fer co-chélateur se trouve dans la gamme comprise entre 0,01:10 et 10:0,01.

12. Composition selon la revendication 11, dans laquelle le rapport molaire du composé de type pyranone au fer co-chélateur se trouve dans la gamme comprise entre 0,01:1 et 1:0,01 et dans laquelle sur la base du poids de la composition, la pyrithione de zinc comprend entre 0,001 % et 5,0 % en poids, ledit composé de type pyranone comprend entre 0,001 % et 5,0 % en poids et ledit fer co-chélateur comprend entre 0,001 % et 5,0 % en poids.
